(19) Europäisches Patentamt
European Patent Office
Office européen des brevets

(11) Publication number: 0 216 397 A2

# (12) EUROPEAN PATENT APPLICATION

(21) Application number: 86200684.8

(51) Int. Cl.[4]: C07D 401/04

(22) Date of filing: 22.04.86

(30) Priority: 28.05.85 US 737964

(43) Date of publication of application: 01.04.87 Bulletin 87/14

(84) Designated Contracting States: AT BE CH DE FR GB IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Semple, Joseph Edward
3400 Polaris Street
Modesto California(US)

(74) Representative: Hunter, Keith Roger Ian et al
4 York Road
London SE1 7NA(GB)

## (54) Process for preparing imidazolinyl compounds.

(57) A process for preparing herbicidal/plant growth regulant 2-(4,4-dialkyl-5-oxo-2-imidazolin-2-yl-3-pyridine (and 3-quinoline) carboxylic acids comprises treating a pyridine (or quinoline) dicarboxylic anhydride with a 2-amino-2,2-dialkylacetamide in an inert solvent and heating the resulting mixture with a strong base, removing water of reaction as it is formed.

EP 0 216 397 A2

Xerox Copy Centre

## PROCESS FOR PREPARING IMIDAZOLINYL COMPOUNDS

This invention relates to a process for preparing certain imidazolinyl compounds which are of interest because of their effects on plants.

European Patent Applications 41623 and 41624 respectively disclose the herbicidal and plant growth regulant activity of 2-(4,4-dialkyl-5-oxo-2-imidazolin-2-yl)-3-pyridine (and 3-quinoline) carboxylic acids of the formula

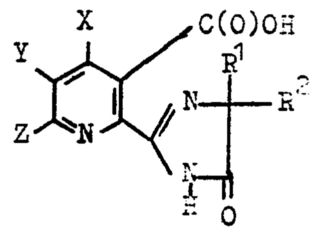

(I)

wherein $R^1$ is $C_{1-4}$ alkyl; $R^2$ is $C_{1-4}$ alkyl or $C_{3-6}$ cycloalkyl; X is hydrogen or $C_{1-4}$ alkyl; Y is hydrogen, halogen, $C_{1-4}$ alkyl, $C_{1-4}$ alkoxy, $C_{1-4}$ alkylthio, trihalomethyl, difluoromethoxy, di($C_{1-4}$ alkyl)-amino, or phenyl or phenoxy either of which may optionally be substituted by one of $C_{1-4}$ alkyl, $C_{1-4}$ alkoxy and halogen; Z is hydrogen, $C_{1-4}$ alkyl, trihalomethyl, or phenyl or phenoxy either of which may optionally be substituted by one of $C_{1-4}$ alkyl, $C_{1-4}$ alkoxy and halogen; or Y and Z together may represent the moiety

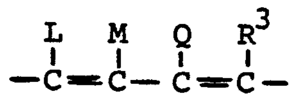

wherein each of L, M, Q and $R^3$ represents hydrogen, halogen, $C_{1-4}$ alkyl, $C_{1-4}$ alkoxy, $C_{1-4}$ alkylthio or $C_{1-4}$ haloalkyl, difluoromethoxy, di($C_{1-4}$ alkyl)amino, nitro, phenyl or phenoxy either of which may optionally be substituted by one of $C_{1-4}$alkyl, $C_{1-4}$ alkoxy and halogen, with the proviso that only one of L, M, Q and $R^3$ may represent a substituent other than halogen, $C_{1-4}$ alkyl or $C_{1-4}$ alkoxy.

When $R^1$ and $R^2$ are different moieties, the carbon to which they are bonded is an asymmetric centre, and the compound exists in the d-and 1-isomeric forms. Further, all of the compounds exist as tautomers, one tautomeric form being represented by Formula I, and the other form being represented by the formula

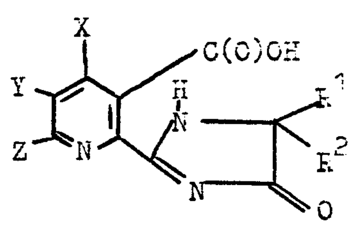

(IA)

For simplicity, both forms are referred to herein and represented by Formula I, and by the generic name set out in the first paragraph and repeated elsewhere herein, including the claims.

According to European Patent Application 95105, such compounds are prepared by effecting reaction between an anhydride of the formula

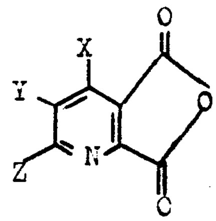

(II)

and an aminocarboxamide of the formula

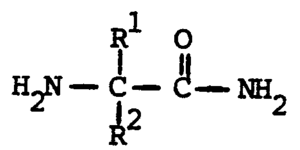

(III)

to form an acid of the formula

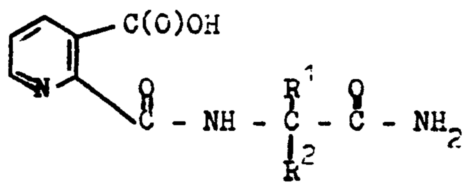

(IV)

which is treated with a base to form the compound of Formula I.

The reaction of the anhydride (II) with the amide (III) is effected by mixing them in an inert solvent at a temperature of 20-80°C. Suitable solvents are disclosed to be ethers, acetonitrile, ethyl acetate and halogenated hydrocarbons. The product (IV) is isolated and then treated with at least two molar equivalents of an aqueous or aqueous/alcoholic solution of sodium or potassium hydroxide containing at least 10% by weight of the hydroxide, at a temperature of 25-100°C - (preferably at reflux), then acidifying the mixture to a pH of 2 to 4, to give the product (I).

According to the present invention compounds of Formula I are prepared by contacting the anhydride (II) with the amide (III) in an inert solvent that forms an azeotrope with water, at a temperature above about 15°C, and when the reaction has been completed, adding a strong base as catalyst, and heating the resulting mixture at a temperature above about 70°C, removing by-product water from the mixture essentially as soon as it is formed.

This new process thus enables preparation of the product acid (I) in a single reaction vessel without isolation of the intermediate acid (IV).

The anhydride (II) and the amide (III) may conveniently be used in essentially equimolar proportions and their reaction is effected by their presence in the solvent at a temperature of from about 15°C to about 80°C for a time sufficient to enable the reaction to go to completion. Suitable as the solvent is any inert liquid that is a solvent for the reagents and intermediate acid (IV), and that forms an azeotrope with water.

The cyclization of the acid (IV) is conducted at a temperature of from about 70°C to about 140°C, but preferably below about 120°C to minimize possible undesirable side-reactions. Since the by-product water must be removed as formed, such is most conveniently accomplished by refluxing the mixture, the water being taken overhead as an azeotrope with the solvent. Effecting reflux at the desired reaction temperature can be accomplished by using a solvent having the appropriate boiling point, or by conducting the reaction under the appropriate pressure.

Preferred solvents are aromatic hydrocarbons and aromatic halogen-substituted hydrocarbons. The amount of solvent to be used is not critical - sufficient should be used to provide a fluid reaction mixture, taking into account the amount involved in the azeotropic removal of the water of reaction.

Suitable as the strong base are the alkali metal hydroxides, alkali metal hydrides, alkali metal oxides, 1,8-diazabicyclo[5.4.0]undec-7-ene (DBU), 1,5-diazabicyclo[3.4.0]non-5-ene, 1,4-diazabicyclo-[2.2.2]octane, tetramethylguanidine, potassium fluoride, quaternary ammonium hydroxides such as trimethylbenzyl ammonium hydroxide, strongly basic ion-exchange resins, and tertiary nitrogen bases, such as pyridine and trialkylamines. The

trialkylamines are preferred, triethylamine being particularly effective. The rate of the reaction appears to be related to the amount of the base. Consequently, as little as a "catalytic amount" -a fraction of a molar equivalent -can be used, but to attain optimum reaction rates, it is desirable to use at least about 0.5 equivalent of the base per equivalent of the acid (IV) reagent. The use of more than about one equivalent of the base per equivalent of the acid does not appear to have significant advantage over the use of a lesser amount of the base.

The examples set out hereinafter describe conduct of the treatment in a preferred manner, by refluxing the reaction mixture at atmospheric pressure, using toluene as solvent, and 0.5 to 1.0 equivalent of triethylamine per equivalent of the acid (IV), removing the water of reaction overhead essentially as soon as it forms.

The product acid (I) is isolated by removing the solvent, treating the residue with an aqueous solution of an alkali metal base, such as sodium hydroxide, extracting the resulting mixture with water-immiscible solvent, such as ethyl acetate, to remove impurities, and obtaining the acid by treating the aqueous solution of the alkali metal salt with a mineral acid, such as hydrochloric acid, and extracting the acid with a water-immiscible solvent such as methylene chloride. The techniques used are described in detail in the examples, hereinafter.

The process of the invention is applied with particular advantage to prepare compounds of formula I above in which $R_1$ is methyl, $R_2$ is methyl, ethyl, isopropyl or cyclopropyl, X is hydrogen, and Y is hydrogen, halogen, $C_{1-4}$ alkyl, $C_{1-4}$ alkoxy, trifluoromethyl or phenyl, Z is hydrogen, $C_{1-4}$ alkyl, trifluoromethyl or phenyl; or Y and Z together represent the moiety

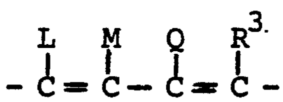

wherein each of L, M, Q and $R^3$ independently represents hydrogen, halogen, methoxy, nitro, $C_{1-4}$ alkyl, trifluoromethyl, dimethylamino or methylthio provided that only one of L, M, Q and $R^3$ may represent a substituent other than halogen, $C_{1-4}$alkyl and methoxy. Preferably, each of Y and Z represents a hydrogen atom, or Y and Z together represent the joint moiety drawn above, in which preferably, L and $R^3$ are both hydrogen, and each of M and Q independently represents hydrogen, halogen, methyl or methoxy, both preferably representing hydrogen.

The following examples illustrate the invention. In each case, the identity of the product and each intermediate involved was confirmed by appropriate chemical and spectral analyses. In neither case was the tautomeric form(s) of the product determined.

Example 1 -2-(5-isopropyl-5-methyl-4-oxo-2-imidazolin-2-yl)-3 quinolinecarboxylic acid (1)

A solution of 58.9 g of ammonium chloride in 140 ml of water was added to a solution of 51.6 g of sodium cyanide in 100 ml of water, then 67 ml of 58% ammonium hydroxide was added, followed by addition, drop-by-drop, of a solution of 86.13 g of 3-methyl-2-butanone in 160 ml of methanol. The mixture was cooled during the addition to maintain its temperature at about 28°C. The mixture then was heated at 57°C for 17 hours, cooled and the methanol was evaporated. The residue was extracted with ether, the extract was washed with aqueous sodium bicarbonate solution and with brine, dried ($MgSO_4$) and stripped of solvent. The residue was distilled through a bantum-ware vigreaux head to give 2-amino-2,3-dimethylbutyronitrile (1A), as an oil, b.p.: 72°C, 1730 Pa.

In a nitrogen atmosphere, 11.2 g of 1A was added drop-by-drop to 25 ml of stirred concentrated sulphuric acid cooled to maintain the mixture at about room temperature. The mixture was stirred for 3 days at room temperature, then poured onto ice, and the resulting mixture was treated with 53 g of sodium carbonate, then with about 5 ml of 50% aqueous sodium hydroxide solution to bring the mixture to a pH of greater than 10. The mixture was filtered, the filtrate was extracted with methylene chloride, the extract was dried ($MgSO_4$) and the solvent was evaporated to give 2-amino-2,3-dimethylbutyramide (1B) as a white solid, m.p.: 76-80°C.

A mixture of 8.14 g of 2,3-quinolinedicarboxylic acid trihydrate and 80 ml of acetic anhydride was stirred and refluxed for 1.5 hours, to give 2,3-quinolinedicarboxylic anhydride, (1F), as a light-brown solid, m.p.: 206-209°C, with decomposition.

3.98 g of 1F, 2.60 g of 1B and 100 ml of toluene were mixed and the mixture was stirred at room temperature for 19 hours. Then 2.02 g of triethylamine was added, the mixture was stirred at room temperature for 30 minutes, then at reflux for 24 hours. Then the mixture was stripped of solvent

and the residue was dissolved in 75 ml of water containing 10% sodium hydroxide, the final mixture having a pH of 9 to 10. The aqueous solution was extracted with ethyl acetate, cooled to 0°C, treated with 6M hydrochloric acid to a pH of 4, stirred at 0°C for 1 hour, then filtered. The resulting solid was collected, washed with water, dried under reduced pressure, and recrystallized from acetone to give 1, as a near colourless solid, m.p.: 235-237°C.

Example 2 -2-(5-isopropyl-5-methyl-4-oxo-2-imidazolin-2-yl)-3-pyridine-3-carboxylic acid (2)

A mixture of 2.98 g of 2,3-pyridinedicarboxylic anhydride and 2.60 g of 1B in 100 ml of toluene was stirred at room temperature for 22 hours. Then 1.01 g of triethylamine was added and the mixture was stirred and refluxed for 17 hours, water being taken overhead as it formed. The mixture was stripped of solvent and the residue was dissolved in 50 ml of water. The solution was made basic to a pH of 9 by adding 10% aqueous sodium hydroxide solution, then was extracted with ethyl acetate. The aqueous phase was acidified to a pH of 3 with 6M hydrochloric acid, then extracted with methylene chloride. The extract was dried - ($MgSO_4$) and stripped of solvent, and the residue was recrystallized from methylene chloride containing a trace of hexane to give 2, as a colourless solid, m.p.: 170-172°C, with decomposition.

## Claims

1. A process for preparing a compound of the formula

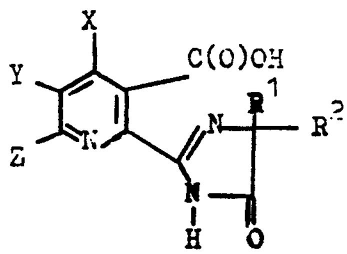

(I)

wherein $R^1$ is $C_{1-4}$ alkyl; $R^2$ is $C_{1-4}$ alkyl or $C_{3-6}$cycloalkyl; X is hydrogen or $C_{1-4}$ alkyl; Y is hydrogen, halogen, $C_{1-4}$ alkyl, $C_{1-4}$ alkoxy, $C_{1-4}$ alkylthio, trihalomethyl, difluoromethoxy, di($C_{1-4}$ alkyl)-amino, or phenyl or phenoxy either of which may optionally be substituted by one of $C_{1-4}$ alkyl, $C_{1-4}$ alkoxy and halogen; Z is hydrogen, $C_{1-4}$ alkyl, trihalomethyl, or phenyl or phenoxy either of which may optionally be substituted by one of $C_{1-4}$ alkyl, $C_{1-4}$ alkoxy and halogen; or Y and Z together may represent the moiety

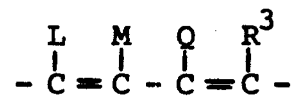

wherein each of L, M, Q and $R^3$ represents hydrogen, halogen, $C_{1-4}$ alkyl, $C_{1-4}$ alkoxy, $C_{1-4}$ alkylthio or $C_{1-4}$haloalkyl, difluoromethoxy, di($C_{1-4}$ alkyl)-amino, nitro, phenyl or phenoxy either of which may optionally be substituted by one of $C_{1-4}$ alkyl, $C_{1-4}$ alkoxy and halogen, with the proviso that only one of L, M, Q and $R^3$ may represent a substituent other than halogen, $C_{1-4}$ alkyl or $C_{1-4}$ alkoxy, which comprises (a) treating an anhydride of the formula

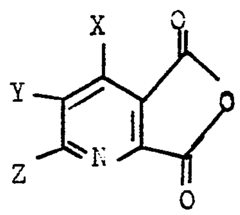

(II)

with an aminocarboxamide of the formula

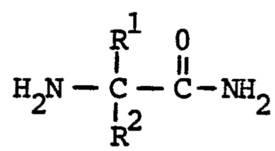

(III)

in an inert solvent that forms an azeotrope with water at a temperature above about 15°C, and (b) when the reaction has gone essentially to completion, adding a strong base as catalyst and heating the resulting mixture at a temperature above about 70°C, recovering by-product water from the mixture essentially as soon as the water is formed.

2. A process according to claim 1 wherein the solvent is an aromatic hydrocarbon or a halogen-substituted aromatic hydrocarbon and in step (b) the reaction mixture is heated at the reflux temperature of the mixture and the water of reaction is removed overhead as an azeotrope with the solvent.

3. A process according to claim 2 wherein the solvent is toluene and the base is triethylamine.